# EUROPEAN PATENT APPLICATION

(11) **EP 1 466 632 A1**
(43) Date of publication of application: **13.10.2004**
(21) Application number: 04252017.1
(22) Date of filing: 02.04.2004
(51) Int. Cl.: A61L 27/36, A61L 27/18, A61F 2/02

(54) **Implantable pouch seeded with insulin-producing cells to treat diabetes**

(30) Priority: 02.04.2003 US 405594
(71) Applicant: Lifescan, Inc., Milpitas, California 95035-6312 (US)
(72) Inventor: Rezania, Alireza, Hillsborough, New Jersey 08448 (US); Zimmerman, Mark, East Brunswick, New Jersey 08816 (US); Ghabrial, Ragae M., Helmetta, New Jersey 08828 (US)
(74) Representative: Mercer, Christopher Paul

(57) **Abstract**

An implantable pouch and methods for implanting cells or cellular matter in mammals, comprising reinforced porous foam and a lumen. The lumen contains an insert that may or may not be removed prior to transplantation. The lumen may be loaded with at least one cell type expressing at least one transcription factor characteristic of a mammalian pancreatic beta cell.

## Description

### FIELD OF THE INVENTION

The present invention relates to an implantable pouch seeded with insulin releasing cells to treat diabetes. More specifically, the present invention provides an implantable porous pouch containing an opening for loading insulin releasing cells to treat diabetes mellitus and which opening may thereafter be closed and, if desired, sealed shut.

### BACKGROUND OF THE INVENTION

Pancreatic tissue consists of three parts: exocrine, endocrine, and ducts. The endocrine pancreas contains islet cells responsible for release of four distinct hormones, and such islets consist of four separate cell types: α, β, δ, and polypeptide cells that produce the hormones glucagons, insulin, somatostatin, and pancreatic polypeptide, respectively. As established in the prior art relating to the identification of endocrine cells, several key transcription factors have been identified which are essential in the development of beta cells including Pdxl, *Ngn3, Hlxb9, Nkx6, Isl1, Pax6, Neurod, Hnf1a, Hnf6* and others. See, for example, Nature *Reviews Genetics,* Vol3, 524-632, 2002.

A common disease of the endocrine pancreas, diabetes mellitus (DM), results from the destruction of beta cells (Type I DM) or from insensitivity of muscle or adipose tissues to the hormone insulin (Type II DM). Current methods of treatment of both Type I and Type II DM includes diet and exercise, oral hypoglycemic agents, insulin injections, insulin pump therapy, and whole pancreas or islet transplantation.

The most common treatment involves daily injections of an endogenous source of insulin such as porcine, bovine, or human insulin. The patient will usually follow a regime involving self-monitoring of blood glucose levels where insulin will be injected according to a prescribed plan based on the results of such blood analysis.

Another, less common, treatment approach has been transplantation of the whole pancreas organ. Such transplants of a whole, adult pancreas are major, technically complex operations which also require aggressive treatment with immunosuppressive drugs to avoid rejection of the newly transplanted organ. Such organs are typically obtained from deceased, human donors, and the limited availability of such cadaver pancreas restricts the widespread use of this approach.

In the transplant field, many have suggested that it would be advantageous to separate the insulin-producing islets from the remainder of the pancreas tissue. Such advantages include less invasive surgery due to the lower tissue mass being transplanted. In addition there would be increased access to immunomanipulation, and engineering of the graft composition.

Until recently, islet grafting has been generally unsuccessful due to aggressive immune rejection of islets. Recent reports (*N. Eng. J*. *Med.* 343:230-238, 2000; Diabetes, 50:710-719, 2001) indicate that a glucocorticoid-free immunosuppressive regimen can significantly benefit the patients with brittle type I diabetes. However, the patients using this treatment are prone to renal complications, mouth ulcers, and require large number of islets (~9000 islet equivalents/kg of patient weight) required to induce normoglycemia. Thus, there has been an intense effort to devise islet cell transplantation strategies that avoid the large doses of immunosuppressive drugs and use a commercially viable islet cell source. This has led to the concept of immunoisolation (Diabetologia, 45:159-173, 2002) which involves shielding of the islets with a selectively permeable membrane. The membrane allows passage of small molecules, such as nutrients, oxygen, glucose, and insulin, while restricting the passage of larger humoral immune molecules and immune cells. In theory, one could use an immunoisolation device with an abundant animal islet cell source, such as porcine, to treat DM. However, in practice this approach has had little success in large animal models or in clinic due to fibrosis of the device, limited oxygen supply within the device, and passage of small humoral immune molecules which lead to islet loss.

An alternative approach to immunoisolation is the creation of an immunologically privileged site by transplanting Sertoli cells into a nontesticular site in a mammal (US 5,849,285, US 6,149,907, US 5,958,404). This site allows for subsequent transplantation of islets that produce insulin. The immune privileged site would allow transplantation of either human or animal derived islets. One of the drawbacks of this approach is that the transplanted Sertoli and islet cells are not physically restricted to site of transplantation. This can lead to migration of these cells to unwanted tissue sites. If the islets migrate away from the Sertoli cells, it could ultimately lead to the loss of islets through loss of the immunosuppressive effect of the Sertoli cells as the immune-privileged environment created by Sertoli cells is most effective when the islets are in Close proximity.

The recent emergence of tissue engineering offers alternative approaches to treat diabetes. Tissue engineering strategies have explored the use of various biomaterials in combination with cells and/or growth factors to develop biological substitutes that ultimately can restore or improve tissue function. For example, scaffold materials have been extensively studied as tissue templates, conduits, barriers, and reservoirs useful for tissue repair. In particular, synthetic and natural materials in the form of foams, sponges, gels, hydrogels, textiles, and nonwovens have been used *in vitro* and *in vivo* to reconstruct and/or regenerate biological tissue, as well as deliver chemotactic agents for inducing tissue growth (US5770417, US6022743, US5567612, US5759830).

One of the key requirements for a scaffold is the retention of cells following seeding onto the scaffold. Until now, scaffolds have been constructed as a substrate material upon which cells, such as islets, are seeded. Traditional porous matrices, such as polygycolic acid nonwovens or polylactic acid foams, though, have a pore size that is either too large or too small to sufficiently retain pancreatic islets or islet-like structures.

Another key requirement for a scaffold loaded with insulin secreting cells is the availability of a functional microvascular bed that allows for exchange of essential nutrients and maintenance of high oxygen tension. Therefore, there remains a need for a three-dimensional construct that can be seeded with a large number of insulin-producing cells, retain the majority of the cells following implantation, and provide a vascular milieu for cell survival. The biodegradable construct of the present invention provides such a three-dimensional Dorous matrix.

### SUMMARY OF THE INVENTION

The present invention is directed to an implantable pouch that is suitable for use in seeding and subsequent implantation of plurality of mammalian cells including insulin-producing cells. In a preferred embodiment, the walls of the pouch are biocompatible and composed of a foam matrix reinforced with a biocompatible mesh. In use, the lumen of the pouch is loaded with an insulin-secreting cell suspension. The biocompatible matrix encapsulating the mesh is preferably porous, polymeric foam, preferably formed using a lyophilization process. The construct may also be used to provide a vascular bed prior to introduction of insulin secreting cells. The lumen of the pouch may be filled with a biocompatible plug, to restrict tissue growth into the lumen, and implanted into a clinically relevant site followed by removal of the plug at a later time and injection of the insulin-secreting cells into the lumen of the pouch. The pouch may be optionally loaded with one or more biologically active compounds or hydrogels. The wall of the pouch preferably is made from a polymer whose glass transition temperature is below physiologic temperature so that the pouch will minimize irritation when implanted in soft tissues.

The construct of the present invention can also act as a'vehicle to deliver cell-secreted biological factors or synthetic pharmaceuticals. Such agents may direct up-regulation or down-regulation of growth factors, proteins, cytokines or proliferation of other cell types. A number of cells may be seeded on such a pouch before or after implantation into a diseased mammal.

### BRIEF DESCRIPTION OF THE FIGURES

Figure 1 shows a perspective drawing of one embodiment of the implantable pouch of the present invention.
Figure 2 is a scanning electron micrograph of one embodiment of the pouch scaffold in the present invention made by the process described in Example 1.
Figure 3 is a perspective drawing of one embodiment of the fabrication process for the implantable pouch described herein.

### DETAILED DESCRIPTION OF THE INVENTION

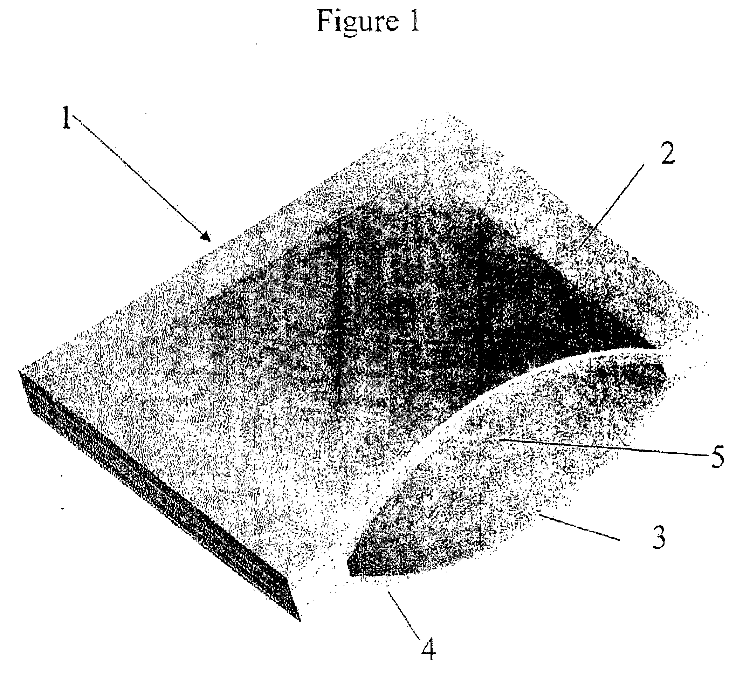

An implantable tissue scaffold pouch is disclosed herein which is used in treatment of diabetes. A perspective view of the implantable tissue scaffold pouch is provided in Figure 1. The implantable pouch 1 consists of a wall 2 surrounding an interior lumen 5. The wall 2 is preferably composed of a porous foam matrix 3 reinforced with, most preferably, a mesh 4. The interior lumen will have a volume of at least 1 x 10⁻³ cm³. Preferably it will be at least 0.1 cm³. The number and size of the insulin-producing cells along with site of implantation will dictate the dimensions of the pouch 1. The porous pouch 1 will generally have a longitudinal axis and a cross-section that may be circular, oval or polygonal. Preferred for ease of manufacture is an oval shaped cross-section.

Figure 1 depicts a pouch constructed from two rectangular sheets sealed on three sides and open at one end. As evident, though, from Figure 1, all that is necessary is a lumen to be formed by the wall 2 such that a cavity is formed sufficient for placement of islets or islet-like cells. Thus, the pouch could be constructed from one sheet or from multiple sheets and sealed in some appropriate manner together.

The walls 2 of the pouch 1 contain pores 6 that may range from about 0.1 to about 500 microns and preferably in the range of from about 5 to about 400 microns. The lumen 5 of implantable pouch 1 may be filled with a hydrogel or a matrix containing a cell suspension or with a non-porous slab of nondegradable material that may be removed at a later time following transplantation and replaced with a cell suspension.

The foam component 3 of the wall 2 is preferably elastomeric, with pore size in the range of 5-400 µm. The foam 3 may be loaded with biologically active or pharmaceutically active compounds (e.g. cytokines (e.g. interlukins 1-18; interferons α, β, and γ; growth factors; colony stimulating factors, chemokines, etc.), non-cytokine leukocyte chemotactic agents (e.g. C5a, LTB₄, etc.), attachment factors, genes, peptides, proteins, nucleotides, anti-inflammatory agents, anti-apoptotic agents, carbohydrates or synthetic molecules.

In the preferred embodiment, the reinforcing component 4 of the wall 2 can be comprised of any absorbable or non-absorbable biocompatible material, including textiles with woven, knitted, warped knitted (i.e., lace-like), non-woven, and braided structures. In an exemplary embodiment, the reinforcing component 4 has a mesh-like structure.

In any of the above structures, mechanical properties of the material can be altered by changing the density or texture of the material, or by embedding particles in the material. The fibers used to make the reinforcing component 4 can be monofilaments, yarns, threads, braids, or bundles of fibers. These fibers can be made of any biocompatible material including bioabsorbable materials such as polylactic acid (PLA), polyglycolic acid (PGA), polycaprolactone (PCL), polydioxanone (PDO), trimethylene carbonate (TMC), polyvinyl alcohol (PVA), copolymers or blends thereof. In one embodiment, the fibers are formed of a polyglycolic acid and polylactic acid copolymer at a 95:5 mole ratio. In another embodiment, the fibers are formed from a 100% PDO polymer.

The wall 2 of the implantable pouch 1 will be made with a biocompatible material that may be absorbable or non-absorbable. The wall 2 will preferably be made from biocompatible materials that are flexible and thereby minimizing irritation to the patient. Preferably the wall 2 will be made from polymers or polymer blends having glass transition temperature below physiologic temperature. Alternatively the pouch can be made with a polymer blended with a plasticizer that makes it flexible.

Numerous biocompatible absorbable and nonabsorbable materials can be used to make the foam component 3. Suitable nonabsorbable materials include, but are not limited to, polyamides, polyesters (e.g. polyethylene terephthalate, polybutyl terphthalate, copolymers and blends thereof), fluoropolymers (e.g. polytetrafluoroethylene and polyvinylidene fluoride, copolymers and blends thereof), polyolefins, polyvinyl resins (e.g. polystyrene, polyvinylpyrrolidone, etc.) and blends thereof.

A variety of bioabsorbable polymers can be used to make the wall 2 of the present invention. Examples of suitable biocompatible and bioabsorbable polymers include but are not limited to polymers selected from the group consisting of aliphatic polyesters, poly(amino acids), copoly(ether-esters), polyalkylenes oxalates, polyamides, tyrosine derived polycarbonates, poly(iminocarbonates), polyorthoesters, polyoxaesters, polyamidoesters, polyoxaesters containing amine groups, poly(anhydrides), polyphosphazenes, biomolecules (i.e., biopolymers such as collagen, elastin, bioabsorbable starches, etc.) and blends thereof.

Particularly well suited for use in the present invention are biocompatible absorbable polymers selected from the group consisting of aliphatic polyesters, copolymers and blends which include but are not limited to homopolymers and copolymers of lactide (which includes D-, L-, lactic acid and D-, L- and meso lactide), glycolide (including glycolic acid), oxaesters, epsilon-caprolactone, p-dioxanone, alkyl substituted derivatives of p-dioxanone (i.e. 6,6-dimethyl-1,4-dioxan-2-one, trimethylene carbonate (1,3-dioxan-2-one), alkyl substituted derivatives of 1,3-dioxanone, delta-valerolactone, beta-butyrolactone, gamma-butyrolactone, epsilon-decalactone, hydroxybutyrate, hydroxyvalerate, 1,4-dioxepan-2-one and its dimer 1,5,8,12-tetraoxacyclotetradecane-7,14-dione, 1,5-dioxepan-2-one, and polymer blends thereof.

The reinforcing component 4 of the wall 2 is preferably composed from lactide and glycolide sometimes referred to herein as simply homopolymers and copolymers of lactide and glycolide and copolymers of glycolide and epsilon-caprolactone, most preferred for use as a mesh is a copolymer that is from about 80 weight percent to about 100 weight percent glycolide with the remainder being lactide. More preferred are copolymers of from about 85 to about 95 weight percent glycolide with the remainder being lactide. Another preferred polymer is 100% PDO.

Preferred foam component 3 is composed of homopolymers, copolymers, or blends of glycolide, lactide, polydioxanone, and epsilon-caproloactone. More preferred are copolymers of glycolide and caprolactone. Most preferred is a 65:35 glycolide:caprolactone copolymer.

As used herein, the term "glycolide" is understood to include polyglycolic acid. Further, the term "lactide" is understood to include L-lactide, D-lactide, blends thereof, and lactic acid polymers and copolymers.

A particularly desirable composition includes an elastomeric copolymer of from about 35 to about 45 weight percent epsilon-caprolactone and from about 55 to about 65 weight percent glycolide, lactide (or lactic acid) and mixtures thereof. Another particularly desirable composition includes para-dioxanone homopolymer or copolymers containing from about 0 to about 80 weight percent para-dioxanone and from about 0 to about 20 weight percent of either lactide, glycolide and combinations thereof. The degradation time for the membrane in-vivo is preferably longer than 1 month but is shorter than 6 months and more preferably is longer than 1 month but less than 4 months.

The molecular weight of the polymers used in the present invention can be varied as is well know in the art to provide the desired performance characteristics. However, it is preferred to have aliphatic polyesters having a molecular weight that provides an inherent viscosity between about 0.5 to about 5.0 deciliters per gram (dl/g) as measured in a 0.1 g/dl solution of hexafluoroisopropanol at 25 °C, and preferably between about 0.7 and 3.5 deciliters per gram (dl/g).

Alternatively, the reinforcing component 4 of the wall 2 can be a nonwoven scaffold. The nonwoven scaffold can be fabricated using wet-lay or dry-lay fabrication techniques. Fusing the fibers of the nonwoven scaffold of the tissue scaffold pouch 1 with another biodegradable polymer, using a thermal process, can further enhance the structural integrity of the fibrous nonwoven scaffold of the tissue scaffold pouch 1. For example, bioabsorbable thermoplastic polymer or copolymer, such as polycaprolactone (PCL) in powder form, may be added to the nonwoven scaffold followed by a mild heat treatment that melts the PCL particles while not affecting the structure of the fibers. This powder possesses a low melting temperature and acts as a binding agent later in the process to increase the tensile strength and shear strength of the nonwoven scaffold. The preferred particulate powder size of PCL is in the range of 10-500 µm in diameter, and more preferably 10-150 µm in diameter. Additional binding agents include a biodegradable polymeric binders selected from the group consisting of polylactic acid, polydioxanone and polyglycolic acid or combinations thereof.

Alternatively, the fibers in the nonwoven scaffold may be fused together by spraying or dip coating the nonwoven scaffold in a solution of another biodegradable polymer.

The foam 3 surrounding the lumen 5 of the present pouch 1 may be formed by a variety of techniques well known to those having ordinary skill in the art. For example, the polymeric starting materials may be foamed by lyophilization, supercritical solvent foaming, gas injection extrusion, gas injection molding or casting with an extractable material (e.g., salts, sugar or similar suitable materials).

In one embodiment, the foam portion 3 of the pouch 1 may be made by a polymer-solvent phase separation technique, such as lyophilization. Generally, however, a polymer solution can be separated into two phases by any one of the four techniques: (a) thermally induced gelation/crystallization; (b) non-solvent induced separation of solvent and polymer phases; (c) chemically induced phase separation, and (d) thermally induced spinodal decomposition. The polymer solution is separated in a controlled manner into either two distinct phases or two bicontinuous phases. Subsequent removal of the solvent phase usually leaves a porous structure of density less than the bulk polymer and pores in the micrometer ranges.

The steps involved in the preparation of the foam component 3 of the wall 2 include choosing the appropriate solvents for the polymers to be lyophilized and preparing a homogeneous solution of the polymer in the solution.
The polymer solution then is subjected to a freezing and a vacuum drying cycle. The freezing step phase-separates the polymer solution and the vacuum drying step removes the solvent by sublimation and/or drying, thus leaving a porous polymer structure, or an interconnected open-cell porous foam.

Suitable solvents that may be used in the preparation of the foam scaffold component 3 include, but are not limited to, tetrahydrofuran (THF), dimethylene fluoride (DMF), and polydioxanone (PDO), p-xylene, N-methyl pyrrolidone, dimethylformamide, chloroform, 1,2-dichloromethane, dimethylsulfoxide and mixtures thereof. Among these solvents, a preferred solvent is 1,4-dioxane. A homogeneous solution of the polymer in the solvent is prepared using standard techniques.

The applicable polymer concentration or amount of solvent that may be utilized will vary with each system. Generally, the amount of polymer in the solution can vary from about 0.01% to about 90% by weight and, preferably, will vary from about 0.05% to about 30% by weight, depending on factors such as the solubility of the polymer in a given solvent and the final properties desired in the foam scaffolding.

When a mesh reinforcing material 4 will be used, it will be positioned between two thin (e.g., 0.4 mm) shims; it should be positioned in a substantially flat orientation at a desired depth in the mold. A metal or Teflon insert that has a cross sectional area corresponding to that required for the pouch 1 is placed between two stretched layers of mesh. The polymer solution is poured in a way that allows air bubbles to escape from between the layers of the mesh component. Preferably, the mold is tilted at a desired angle and pouring is effected at a controlled rate to best prevent bubble formation. One of ordinary skill in the art will appreciate that a number of variables will control the tilt angle and pour rate. Generally, the mold should be tilted at an angle of greater than about 1 degree to avoid, bubble formation. In addition, the rate of pouring should be slow enough to enable any air bubbles to escape from the mold, rather than to be trapped in the mold.

If a mesh material is used as the reinforcing component 4, the density of the mesh openings is an important factor in the formation of a resulting tissue implant with the desired mechanical properties. A low density, or open knitted mesh material, is preferred. One particularly preferred material is a 90/10 copolymer of PGA/PLA, sold under the tradename VICRYL (Ethicon, Inc., Somerville, NJ). One exemplary low density, open knitted mesh is Knitted VICRYL VKM-M, available from Ethicon, Inc., Somerville, NJ. Other knitted or woven mesh material that may be used in the pouch are 95/5 copolymer of PLA/PGA, sold under the tradename PANACRYL (Ethicon, Inc., Somerville, NJ), or 100% PDO polymer.

The mammalian cells loaded into the lumen 5 of the pouch 1 may be isolated from pancreatic tissue including the exocrine, endocrine, and ductal components of the pancreas. Alternatively, minced pancreatic tissue or ductal fragments may be loaded into the lumen 5 of the pouch 1. Furthermore, the cells may be cultured under standard culture conditions to expand the number of cells followed by removal of the cells from culture plates and administering into the device prior to implantation. Alternatively, the isolated cells may be injected directly into the pouch 1 and then cultured under conditions which promote proliferation and deposition of the appropriate biological matrix prior to *in vivo* implantation. In the preferred embodiment, the isolated cells are injected directly into the pouch 1 with no further *in vitro* culturing prior to *in vivo* implantation. In another embodiment, the cells are seeded into another porous biocompatible matrix, such as a nonwoven mat, a hydrogel, or combination thereof, followed by placement into the lumen 5 of the pouch.

Cells that can be seeded or cultured on the construct of the current invention include, but are not limited to cells expressing at least one characteristic marker of a pancreatic beta cell. The cells can be seeded into the lumen 5 of the pouch of the present invention for a short period of time (< 1 day) just prior to implantation, or cultured for longer (> 1 day) period to allow for cell proliferation and matrix synthesis within the pouch 1 prior to implantation.

For treatment of a disease such as diabetes mellitus (DM), the cell-seeded scaffold pouch 1 may be placed in a clinically convenient site such as the subcutaneous space, the mesentery, or the omentum. In this particular case, the pouch 1 of the present invention will act as a vehicle to entrap the administered cells in place after *in vivo* transplantation into an ectopic site.

Previous attempts in direct transplantation of islets through injection into the portal circulation has proven inadequate in long-term treatment of diabetes.
Furthermore, numerous methods of encapsulation of allogeneic or xenogeneic beta cells with biodegradable or nondegradable microspheres have failed to sustain long-term control of blood glucose levels. These failures have been attributed to inadequate vasculature and/or immune rejection of transplanted islets.

The failures can be circumvented by administering xenogeneic or allogeneic insulin-producing cells in combination with allogeneic or xenogeneic Sertoli cells which may aid in the survival of the islets and prevention of an immune response to the transplanted islets. Xenogeneic, allogeneic, or transformed Sertoli cells can protect themselves in the kidney capsule while immunoprotecting allogeneic or xenogeneic islets.

In another alternative embodiment of the invention, the wall 2 of the pouch 1 may be modified either through physical or chemical means to contain biological or synthetic factors that promote attachment, proliferation, differentiation, and matrix synthesis of targeted cell types. Furthermore, the bioactive factors may also comprise part of the matrix for controlled release of the factor to elicit a desired biological function. Another embodiment would include delivery of small molecules that affect the up or down regulation of endogenous growth factors. Growth factors, extracellular matrix proteins, and biologically relevant peptide fragments that can be used with the matrices of the current invention include, but are not limited to, members of TGF-β family, including TGF-β1, 2, and 3, bone morphogenic proteins (BMP-2, -4, 6, -12, -13 and -14), fibroblast growth factors-1 and -2, platelet-derived growth factor-AA, and -BB, platelet rich plasma, insulin growth factor (IGF-I, II) growth differentiation factor (GDF-5, -6, -8, -10), angiogen, erythropoiethin, placenta growth factor, angiogenic factors such as vascular endothelial cell-derived growth factor (VEGF), cathelicidins, defensins, glucacgon-like peptide I, exendin-4, pleiotrophin, endothelin, parathyroid hormone, stem cell factor, colony stimulating factor, tenascin-C, tropoelastin, thrombin-derived peptides, anti-rejection agents; analgesics, anti-inflammatory agents such as acetoaminophen, anti-apoptotic agents, statins, cytostatic agents such as Rapamycin and biological peptides containing cell- and heparin-binding domains of adhesive extracellular matrix proteins such as fibronectin and vitronectin. The biological factors may be obtained either through a commercial source, isolated and purified from a tissue or chemically synthesized.

### EXAMPLES

The following examples illustrate the construction of a pouch for implanting cells and cellular matter in mammals. Those skilled in the art will realize that these specific examples do not limit the scope of this invention and many alternative forms of a pouch 1 could also be generated within the scope of this invention.

### EXAMPLE 1: Fabrication of an Implantable Pouch

A solution of the polymer to be lyophilized into a pouch was prepared. The polymer used to manufacture the foam component was a copolymer of 35% PCL and 65% PGA (35/65 PCL/PGA) produced by Birmingham Polymers Inc. (Birmingham, AL) with an I.V. of 1.79 dL/g, as measured in HFIP at 30 °C. A 95/5 weight ratio of 1,4-dioxane/(35/65 PCL/PGA) was weighed out. The polymer and solvent were placed into a flask, which in turn was put into a water bath and stirred at 70°C for 5 hrs. The solution was filtered using an extraction thimble (extra coarse porosity, type ASTM 170-220 (EC)) and stored in a flask.

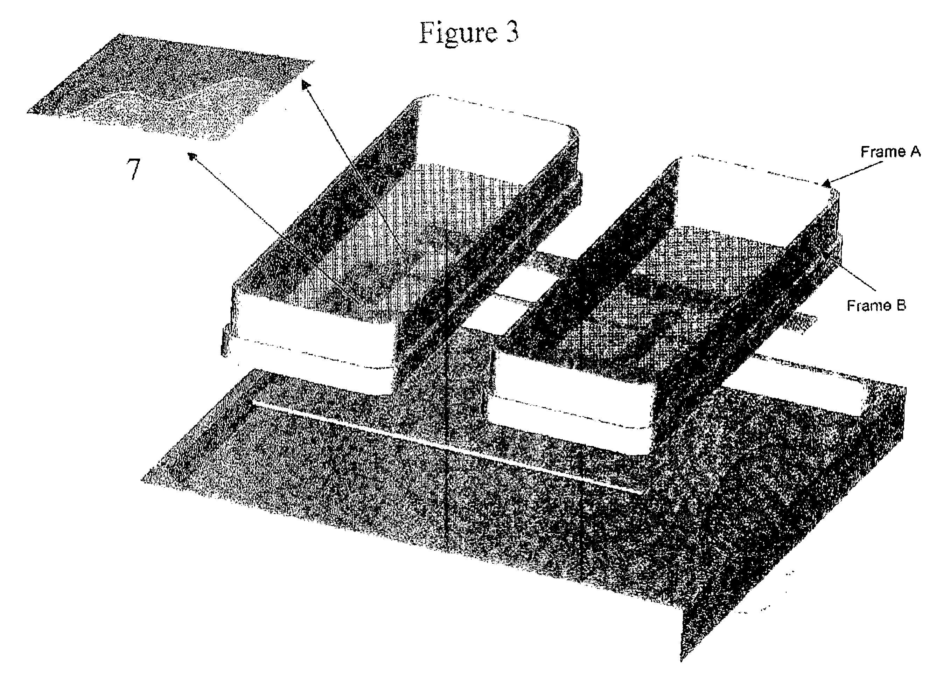

Reinforcing mesh material formed of a 90/10 copolymer of polyglycolic/polylactic acid (PGA/PLA) knitted (Code VKM-M) mesh sold under the tradename VICRYL were rendered flat by ironing, using a compression molder at 80 °C/2 min. After preparing the meshes, 0.4-mm shims were placed at each end of a 15.3 ×15.3 cm aluminum mold, and two meshes were sized to fabricate the desired pouch size. The two mesh layers were stretched on top of each other between frame A and B as indicated in Figure 3 and the complex was then positioned on the shims allowing the meshes to be suspended in solution to be added. A metal or a Teflon insert that has a cross sectional area corresponding to that of the opening of the required pouch (0.4x8.0 or 0.4x4.0 mm²) is placed between two stretched layers of mesh. The polymer solution heated to 50°C is poured slowly from the side until the top mesh layer is completely covered. Approximately 60 ml of the polymer solution was slowly transferred into the mold, ensuring that the solution was well dispersed in the mold. The mold was then placed on a shelf in a Virtis, Freeze Mobile G freeze dryer. The freeze dry sequence used in this example was: 1) -17°C for 60 minutes; 2) -5°C for 60 minutes under vacuum 100 mT; 3) 5°C for 60 minutes under, vacuum 20 mT; 4) 20°C for 60 minutes under vacuum 20 mT.

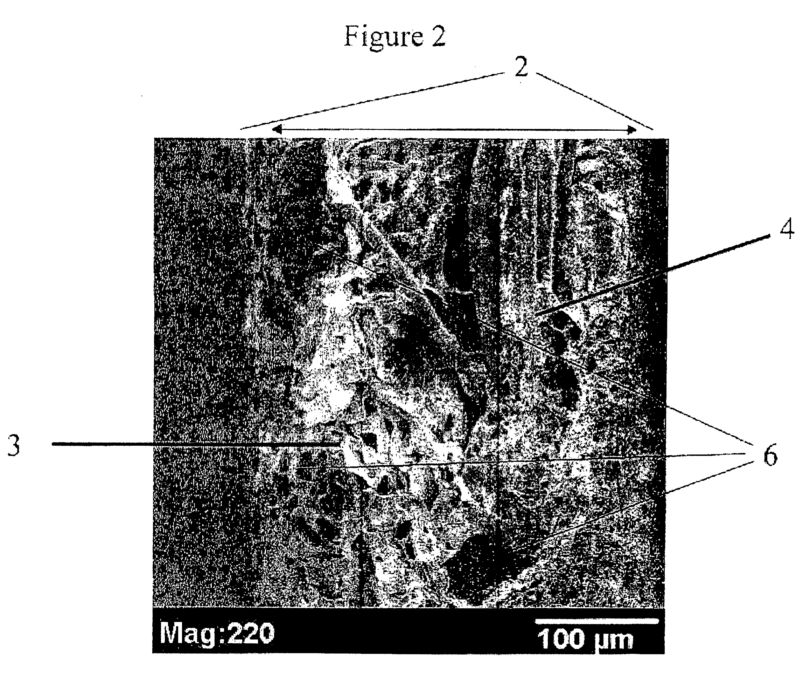

Figure 1 shows the resulting pouch containing the reinforced foam 3 surrounding the lumen of the pouch following the removal of the insert. Figure 2 depicts scanning electron micrograph (SEMs) of the cross-section of the pouch. The SEM clearly shows the lyophilized reinforced foam scaffold inside the pouch. The mold assembly was then removed from the freezer and placed in a nitrogen box overnight. Following the completion of this process the resulting construct was carefully peeled out of the mold in the form of a foam/mesh sheet containing a removable insert. The insert may be removed prior to loading of cells and in vivo implantation or removed at a later time following transplantation. In the latter case, cells are loaded into the lumen of the pouch upon removal of the insert.

### EXAMPLE 2: Fabrication of an Implantable Pouch

A biodegradable pouch was fabricated following the process of Example 1, except a woven Vicryl (Code VWM-M), reinforcing mesh material formed of a 90/10 copolymer of polyglycolic/polylactic acid (PGA/PLA) was used.

### EXAMPLE 3: Fabrication of an Implantable Pouch

A biodegradable pouch was fabricated following the process of Example 1, except a knitted reinforcing mesh material formed of 100% PDS was used.

### EXAMPLE 4: IMPLANTABLE TISSUE SCAFFOLDS WITH MAMMALIAN CELLS

This example illustrates seeding of murine islets within the lumen of the pouch described in this invention.

Murine Islets were isolated from Balb/c mice by collagenase digestion of the pancreas and Ficoll density gradient centrifugation followed by hand picking of islets.

Pouches were prepared as described in Example 1 and seeded with 500 fresh islets and cultured for 1 week in Hams-F10 (Gibco Life Technologies, Rockville, MD) supplemented with bovine serum albumin (0.5%), nicotinamide (10 mM), D-glucose (10 mM), L-glutamine (2 mM), IBMX (3-Isobutyl-1-methylxanthine, 50 mM), and penicillin/Streptomycin. Following 1 week, the islets residing in the pouches were stained with calcein and ethidium homodimer (Molecular Probes, Oregon) to assay for viability of the seeded cells. Majority of the islets stained positive for calcein indicating viable cells within the lumen of the pouch.

## Claims

1. An implantable pouch comprising a biocompatible wall and a lumen, wherein the wall has a plurality of pores of suitable size to allow the ingress and egress of cells and nutrients of a particular size and not allow the ingress and egress of cells of a size larger than the particular size.

2. The pouch of claim 1 wherein the pore size is from 0.1 to 500 microns.

3. The pouch of claim 2 wherein the pore size is from 5 to 400 microns.

4. The pouch of any one of claims 1 to 3 wherein the lumen has a capacity of at least about 1 x 10⁻³ cm⁻³.

5. The pouch of claim 4 wherein the lumen has a capacity of at least about 0.1 cm³.

6. The pouch of any one of claims 1 to 5 further comprising a reinforcing component.

7. The pouch of claim 6 wherein the reinforcing component is a mesh.

8. The pouch of claim 7 wherein the mesh reinforcing component is a homopolymer or copolymer of lactide and glycolide or of glycolide and epsilon-caprolactone.

9. The pouch of claim 8 wherein the mesh reinforcing component is from 80 percent to 100 weight percent glycolide with the remainder being lactide.

10. The pouch of any one of claims 1 to 9 wherein the wall is made of a biocompatible material.

11. The pouch of claim 10, wherein the biocompatible material is a polymer.

12. The pouch of claim 11 wherein the polymer is a homopolymer, copolymer or blend of glycolide, lactide, polydioxanone or epsilon-caprolactone.

13. The pouch of claim 12 wherein the polymer is a copolymer of glycolide and caprolactone.

14. The pouch of any one of claims 1 to 13 wherein the wall comprises a foam.

15. The pouch of claim 14 wherein the foam is impregnated with a biocompatible active agent.

16. The pouch of any one of claims 1 to 15 wherein the lumen is filled with material containing insulin-producing cells.

17. The pouch of claim 16 wherein the lumen also contains Sertoli cells.

18. The pouch of any one of claims 1 to 17 for use in therapy, in particular for the treatment of diabetes mellitus.

19. A method of making a pouch according to claim 11 or any claim dependent thereon comprising selecting a polymer, lyophilizing the polymer and forming the resulting lyophilized polymer into an envelope.
